# EUROPEAN PATENT APPLICATION

(11) **EP 1 205 189 A2**
(43) Date of publication of application: **15.05.2002**
(21) Application number: 01309413.1
(22) Date of filing: 06.11.2001
(51) Int. Cl.: A61K 45/06, A61P 19/00

(54) **Combination of a prostaglandin agonist and a HMG-CoA reductase inhibitor for the stimulation of bone growth**

(30) Priority: 07.11.2000 US 246453 P
(71) Applicant: Pfizer Products Inc., Groton, Connecticut 06340 (US)
(72) Inventor: Paralkar, Vishwas Madhav, Groton, Connecticut 06340 (US)
(74) Representative: Hayles, James Richard

(57) **Abstract**

This invention relates to pharmaceutical combinations of a prostaglandin agonist and a HMG-CoA reductase inhibitor, methods of using such combinations and kits containing such combinations. The pharmaceutical combinations, methods and kits are useful to enhance bone formation in mammals, including humans.

## Description

### BACKGROUND OF THE INVENTION

This invention relates to pharmaceutical combinations of a prostaglandin agonist and a HMG-CoA reductase inhibitor, kits containing such combinations and the use of such combinations to enhance bone formation in mammals, including humans.

There exist many conditions characterized by the need for enhanced bone formation. One condition that is prevalent is osteoporosis. Osteoporosis is a systemic skeletal disease characterized by low bone mass and deterioration of bone tissue with a consequent increase in bone fragility and susceptibility to fracture. In the U.S., the condition affects more than 25 million people and causes more than 1.3 million fractures each year, including 500,000 spine, 250,000 hip and 240,000 wrist fractures. Hip fractures are the most serious with 5-20% of patients dying within one year and over 50% of survivors being incapacitated.

Other such conditions include bone fractures, periodontal disease, metastatic bone disease, and conditions which benefit from plastic surgery and facial and bone reconstruction procedures.

There are currently two main types of pharmaceutical therapies for the treatment of osteoporosis and skeletal fractures. The first is the use of anti-resorptive compounds to inhibit the resorption of bone tissue, thereby preventing bone loss and reducing the incidence of skeletal fractures.

Estrogen is an example of an anti-resorptive agent. It is known that estrogen prevents post-menopausal bone loss and reduces skeletal fractures.

Bisphosphonates are also anti-resorptive agents used to combat osteoporosis. One such biphosphonate is Fosamax® (alendronate). Others include risedronate, tiludronate, and ibandronate.

However, neither estrogen nor biphosphonates stimulate the formation of bone. Also, long-term estrogen therapy has been implicated in a variety of disorders, including an increase in the risk of uterine cancer, endometrial cancer and possibly breast cancer.

A second type of pharmaceutical therapy for the treatment of osteoporosis and bone fractures is the use of anabolic agents. These include prostaglandins and prostaglandin receptor agonsts. Prostaglandin type compounds useful for bone formation are disclosed in U.S. Patent 3,932,389, U.S. Patent 3,982,016, U.S. Patent 4,000,309, U.S. Patent 4,018,892, U.S. Patent No. 4,097,601, U.S. Patent 4,132,847, U.S. Patent 4,219,483,U.S. Patent 4,621,100, U.S. Patent 5,216,183, U.S. Patent No. 5,703,108, U.S. Patent 6,124,314, WO 97/31640, WO 98/27976, WO 98/28264, WO 98/58911 and WO 99/19300. The disclosure of all the foregoing patents and published patent applications are incorporated herein by reference.

The natural prostaglandin, PGE₂, has been reported to stimulate bone formation and increase bone mass and bone strength. EP₁, EP₂, EP₃ and EP₄ are receptors of PGE₂. WO 98/27976, incorporated herein by reference, discloses a method for preventing bone loss and augmenting bone mass using selective EP₂ receptor subtype agonists.

Statins are 3-hydroxy-3-methylglutaryl coenzyme A (HMG-CoA) reductase inhibitors, a class of cardiovascular drugs known to be effective in lowering blood plasma cholesterol levels. Lipitor® (atorvastatin calcium) is one such HMG-CoA reductase inhibitor. HMG-CoA reductase inhibitors include such compounds as simvastatin, disclosed in U.S. 4,444,784, pravastatin, disclosed in U.S. 4,346,227, cerivastatin, disclosed in U.S. 5,502,199, mevastatin, disclosed in U.S. 3,983,140, velostatin, disclosed in U.S. 4,448,784 and U.S. 4,450,171, fluvastatin, disclosed in U.S. 4,739,073, lovastatin, disclosed in U.S. 4,231,938, dalvastatin, disclosed in European Patent Application Publication No. 738,510 A2, fluindostatin, disclosed in European Patent Application Publication No. 363,934 A1; atorvastatin, disclosed in U.S. Patent No. 4,681,893, and atorvastatin calcium, disclosed in U.S. Patent No. 5,273,995, all of which are incorporated herein by reference.

Statins have recently been shown to be effective for stimulating bone formation. G. Mundy, et al., Stimulation of Bone Formation in Vitro and in Rodents by Statins, Science, 286, 1946 (1999). HMG-CoA reductase inhibitors are considered especially useful in that they not only increase the formation of new bone, but also enhance the accumulation of mature osteoblasts, the cells involved in new bone growth.

U.S. Patent No. 6,022,877 and U.S. Patent No. 6,080,799, both incorporated herein by reference, disclose the use of statin compounds for stimulating bone growth.

WO 99/45923 A1, incorporated herein by reference discloses the use of HMG-CoA reductase inhibitors as a therapy for bone conditions. The HMG-CoA reductase inhibitors are described in the published PCT application as having properties related to the inhibition of bone resorption rather than stimulation of bone growth.

Combination therapies have been proposed which combine a bone cell activating agent and a bone resorption inhibiting agent.

EP 0381296 A1, which is incorporated herein by reference, describes the use of a bone cell activating compound in combination with a bone resorption inhibiting polyphosphonate for treatment or prevention of osteoporosis.

WO94/06750 discloses the simultaneous delivery of a bone activating agent such as a prostaglandin chemically coupled to a bone resorption inhibiting compound.

The aforementioned publication WO 99/45923 A1 discloses the administration of a HMG-CoA reductase inhibitor with an agent selected from organic bisphosphonates, estrogen receptor modulators and peptide hormones for inhibition of bone resorption.

The aforementioned U.S. Patent No. 6,080,779 discloses combination therapies for stimulation of bone growth of statin compounds with compounds of the general formula

Ar¹―L―Ar²

wherein Ar¹ and Ar² are independently substituted or unsubstituted phenyl, naphthyl, or a 5 or 6 member aromatic system and L is a linker which spaces Ar¹ and Ar² at a distance of 1.5-15 Å as well as with estrogen compounds and biphosphonates.

No combination therapies are known or are suggested that combine HMG-CoA reductase inhibitors with prostaglandin receptor agonists. Although there are a variety of therapies relating to conditions affecting bone such as osteoporosis, bone fracture healing and bone growth and re-growth, there is a continuing need and a continuing search in this field of art for alternative therapies.

### SUMMARY OF THE INVENTION

One aspect of the present invention are pharmaceutical compositions for promoting bone growth comprising a therapeutically effective amount of a first compound, said first compound being a prostaglandin agonist, or a prodrug thereof or a pharmaceutically acceptable salt of said compound or said prodrug; and a therapeutically effective amount of a second compound, said second compound being a HMG-CoA reductase inhibitor, or a prodrug thereof or a pharmaceutically acceptable salt of said compound or said prodrug.

Another aspect of this invention are methods of promoting bone growth comprising administering to a mammal:
a therapeutically effective amount of a first compound, said first compound being a prostaglandin agonist, or a prodrug thereof or a pharmaceutically acceptable salt of said compound or said prodrug; and
a therapeutically effective amount of a second compound, said second compound being a HMG-CoA reductase inhibitor, or a prodrug thereof or a pharmaceutically acceptable salt of said compound or said prodrug.

A further aspect of the invention are kits comprising:
a. an amount of a first compound, said first compound being a prostaglandin agonist, or a prodrug thereof or a pharmaceutically acceptable salt of said compound or said prodrug in a first dosage form;
b. an amount of a second compound, said second compound being a HMG-CoA reductase inhibitor, or a prodrug thereof or a pharmaceutically acceptable salt of said compound or said prodrug in a second dosage form; and
c. a container.

In a preferred embodiment of the aspects of this invention, said first compound is selected from PGD₁, PGD₂, PGE₂, PGE₁, PGF₂ or PGF_{2α}.

In another preferred embodiment, the first compound is selected from a selective EP₁, EP₂, EP₃ or EP₄ agonist.

In a more preferred embodiment said first compound is selected from a selective EP₂ agonist, a selective EP₄ agonist or an EP₂/EP₄ agonist.

In a still more preferred embodiment, said first compound is selected from: 2-(3-{[2-(3,5-dichloro-phenoxy)-ethyl]-methanesulfonyl-amino}-propyl)-thiazole-4-carboxylic acid; 2-(3-{[3-(3-chloro-phenyl)-propyl]-methanesulfonyl-amino}-propyl)-thiazole-4-carboxylic acid; (3-(((4-tert-butyl-benzyl)-(pyridine-3-sulfonyl)-amino)-methyl)-phenoxy)-acetic acid; (3-(((2-(3,5-dichloro-phenoxy)-ethyl)-(pyridine-3-sulfonyl)-amino)-methyl)-phenoxy)-acetic acid; (3-(((4-dimethylamino-benzyl)-(pyridine-3-sulfonyl)-amino)-methyl)-phenoxy)-acetic acid; or 7-[(4-butylbenzyl)-methanesulfonyl-amino]-heptanoic acid.

In another preferred embodiment, said second compound is selected from mevastatin, lovastatin, pravastatin, velostatin, simvastatin, fluvastatin, cerivastatin and mevastatin, dalvastatin and fluindostatin and atorvastatin, or a prodrug thereof, or a pharmaceutically acceptable salt of said compound or prodrug.

In a still more preferred embodiment, the second compound is atorvastatin, most preferably atorvastatin calcium.

In another preferred embodiment of the method for promoting bone growth, the mammal is a human.

The term "prostaglandin agonist" refers to a compound which binds to the prostaglandin receptors (e.g., An S. et al., Cloning and Expression of the EP₂ Subtype of Human Receptors for Prostaglandin E₂, Biochemical and Biophysical Research Communications, 1993, 197(1):263-270) and mimics the action of prostaglandin in vivo (e.g., stimulates bone formation and increases bone mass). Such compounds include natural prostaglandins such as PGD₁, PGD₂, PGE₂, PGE₁, PGF₂ and PGF_{2α}, and analogs of the natural prostaglandins, including compounds which bind to the PGE₂ receptors, EP₁, EP₂, EP₃ and EP₄. The binding of prostaglandin agonists to prostaglandin receptors is readily determined by those skilled in the art employing standard assays. The bone stimulating activity of prostaglandins is readily determined by those skilled in the art according to standard assays (e.g., see Eriksen E.F. et al., Bone Histomorphometry, Raven Press, New York, 1994, pages 1-74; Grier S.J. et. al., The Use of Dual-Energy X-Ray Absorptiometry In Animals, Inv. Radiol., 1996, 31(1):50-62; Wahner H.W. and Fogelman I., The Evaluation of Osteoporosis: Dual Energy X-Ray Absorptiometry in Clinical Practice., Martin Dunitz Ltd., London 1994, pages 1-296). Prostaglandin agonists include the compounds disclosed in U.S. Patent 3,932,389, U.S. Patent 3,982,016, U.S. Patent 4,000,309, U.S. Patent 4,018,892, U.S. Patent No. 4,097,601, U.S. Patent 4,132,847, U.S. Patent4,219,483, U.S. Patent 4,621,100, U.S. Patent 5,216,183, U.S. Patent No. 5,703,108, U.S. Patent 6,124,314, WO 97/31640, WO 98/27976, WO 98/28264, WO 98/58911 and WO 99/19300.

The term "selective EP₂ agonist" refers to a compound that binds to the EP₂ receptor preferentially (by at least 5 fold) over the EP₁, EP₃ and EP₄ receptors. The term "selective EP₁ agonist" refers to a compound that binds to the EP₁ receptor preferentially (by at least 5 fold) over the EP₂, EP₃ and EP₄ receptors. The term "selective EP₃ agonist" refers to a compound that binds to the EP₃ receptor preferentially (by at least 5 fold) over the EP₁, EP₂ and EP₄ receptors. The term "selective EP₄ agonist" refers to a compound that binds to the EP₄ receptor preferentially (by at least 5 fold) over the EP₁, EP₂ and EP₃ receptors.

The term "EP₂/EP₄ agonist" refers to a compound that binds to both the EP₂ and the EP₄ receptor.

The term "HMG-CoA reductase inhibitor" is intended to include compounds which inhibit the enzyme 3-hydroxy-3-methylglutaryl coenzyme A (HMG-CoA) reductase. HMG-CoA reductase inhibitors includes the compounds known as statins which include mevastatin, lovastatin, pravastatin, velostatin, simvastatin, fluvastatin, cerivastatin and mevastatin, dalvastatin and fluindostatin and atorvastatin and pharmaceutically acceptable salts thereof.

The term "treating", "treat" or "treatment" as used herein includes preventative (e.g., prophylactic) and palliative treatment.

The expression "prodrug" refers to compounds that are drug precursors which, following administration, release the drug *in vivo* via some chemical or physiological process (e.g., a prodrug on being brought to the physiological pH or through enzyme action is converted to the desired drug form).

The expression "pharmaceutically acceptable" refers to carriers, diluents, excipients, and/or salts that are compatible with the other ingredients of the formulation and are not deleterious to the recipient thereof.

The expression "pharmaceutically-acceptable salt" refers to nontoxic anionic salts containing anions such as (but not limited to) chloride, bromide, iodide, sulfate, bisulfate, phosphate, acetate, maleate, fumarate, oxalate, lactate, tartrate, citrate, gluconate, methanesulfonate and 4-toluene-sulfonate. The expression also refers to nontoxic cationic salts such as (but not limited to) sodium, potassium, calcium, magnesium, ammonium or protonated benzathine (N,N'-dibenzylethylenediamine), choline, ethanolamine, diethanolamine, ethylenediamine, meglamine (N-methyl-glucamine), benethamine (N-benzylphenethylamine), piperazine or tromethamine (2-amino-2-hydroxymethyl-1,3-propanediol).

### DETAILED DESCRIPTION OF THE INVENTION

Representative uses of the combination therapy of the present invention include: treatment of skeletal disorders, such as age-related osteoporosis, post-menopausal osteoporosis, glucocorticoid-induced osteoporosis or disuse osteoporosis and arthritis, elevation of peak bone mass in pre-menopausal women, repair of bone defects and deficiencies, including bone fractures, promotion of bone healing in plastic surgery; stimulation of bone ingrowth into non-cemented prosthetic joints and dental implants, treatment of growth deficiencies and treatment of periodontal disease and defects,. Other uses include repair of congenital, trauma-induced or surgical resection of bone. Further, the combination therapies of the present invention can be used for limiting or treating cartilage defects or disorders, and may be useful in wound healing.

Any prostaglandin agonist may be used as the first compound of this invention. Specific prostaglandin agonist compounds are described and referenced below. However, other prostaglandin agonists will be known to those skilled in the art. PCT application publication number WO 98/27976 discloses an in vivo assay in rats to test for activity of anabolic bone agents. Such an assay may readily be used to screen for prostaglandin agonist type compounds having bone formation activity.

Four different subtypes of PGE₂ receptors (EP₁, EP₂, EP₃ and EP₄) have been cloned (Funk, C. D., et al., Cloning and Expression of a cDNA for the Human Prostaglandin E Receptor EP₁ Subtype, Journal of Biological Chemistry, vol. 268, No. 35, pp. 26767-26772,1993; Regan, J. W., et al., Cloning of a Novel Human Prostaglandin Receptor with Characteristics of the Pharmacologically Defined EP₂ Subtype, Molecular Pharmacology, vol. 46, pp. 213-220, 1994; Yang, J., et al., Cloning and Expression of the EP₃-Subtype of Human Receptors for Prostaglandin E₂, Biochemical Biophysical Research Communication, vol. 198, pp. 999-1006, 1994; Bastien, L., et al., Cloning, Functional Expression and Characterization of the Human Prostaglandin E₂ Receptor EP₂ Subtype, Journal Biological Chemistry, vol. 269, pp. 11873-11877, 1994). J. Bone Miner. Res. 1996, 11 (supp.):SI 74 discuss the different subtypes of the PGE₂ receptors.

The binding of prostaglandin agonists to prostaglandin receptors is readily determined by those skilled in the art employing standard assays. PCT application publication number WO 98/27976 discloses an assay for binding to prostoglandin PGE₂ type receptors.

Any HMG-CoA reductase inhibitor may be used as the second compound of this invention, including mevastatin, lovastatin, pravastatin, velostatin, simvastatin, fluvastatin, cerivastatin, mevastatin, dalvastatin, fluindostatin and atorvastatin, or a pharmaceutically acceptable salt thereof.

Statins enhance the production of osteoblasts, the cells that produce new bone. The expression of the bone growth factor Bone Morphogenetic Protein (BMP) is known to enhance osteoblast differentiation. S.E. Harris, et al., Mol. Cell. Differ. 3, 137 (1995). Statins are in turn found to enhance BMP production. G. Mundy, et al., Stimulation of Bone Formation in Vitro and in Rodents by Statins, Science, 286, 1946 (1999). Mundy, et al. find that statins increase new bone formation as well as increase osteoblast cell numbers at all stages of differentiation.

### Assay for Compounds that Enhance BMP-2 Expression

Cultured murine (2T3) or human (MG-63) bone cells exposed to statins show an enhanced expression of the mRNA for BMP-2, one of the BMP's as described in G. Mundy, et al., Stimulation of Bone Formation in Vitro and in Rodents by Statins, Science, 286, 1946 (1999). U.S. Patent 6,080,779 describes an assay used for screening compounds that enhance BMP-2 expression using immortalized mouse cells transfected with a plasmid containing a luciferase reporter gene driven by a mouse BMP2 promoter.

### Protocol for Effects on Fracture Healing After Administration of Combinations of Prostaglandin Agonist and HMG-Co Reductase Inhibitor ― Rat Model

Fracture Technique: Sprage-Dawley rats at 3 months of age are anesthetized with ketamine. A 1 cm incision is made on the anteromedial aspect of the proximal part of the right tibia or femur. The following describes the tibial surgical technique. The incision is carried through to the bone, and a 1 mm hole is drilled 4 mm proximal to the distal aspect of the tibial tuberosity 2 mm medial to the anterior ridge. Intramedullary nailing is performed with a 0.8 mm stainless steel tube (maximum load 36.3 N, maximum stiffness 61.8 N/mm, tested under the same conditions as the bones). No reaming of the medullary canal is performed. A standardized closed fracture is produced 2 mm above the tibiofibular junction by three-point bending using specially designed adjustable forceps with blunt jaws. To minimize soft tissue damage, care is taken not to displace the fracture. The skin is closed with monofilament nylon sutures. The operation is performed under sterile conditions. Radiographs of all fractures are taken immediately after nailing, and animals with fractures outside the specified diaphyseal area or with displaced nails are excluded. The remaining animals are divided randomly into the following groups with 10 - 12 animals per each subgroup for testing the fracture healing.

For systemic administration, the first group receives daily gavage of carrier of the prostaglandin agonist and carrier of the HMG-CoA reductase inhibitor at 1 ml/rat. The second group receives daily gavage of carrier containing each of the prostaglandin agonist and HMG-CoA reductase inhibitor to be tested (1 ml/rat) for 10, 20, 40 and 80 days.

For local administration, the first group receives carrier for the prostaglandin agonist and carrier for the HMG-CoA reductase inhibitor by injection directly in the defect area. The second group receives carrier containing the combination prostaglandin agonist and the HMG-CoA reductase inhibitor to be tested.

At 10, 20, 40 and 80 days, 10 - 12 rats from each group are anesthetized with ketamine and autopsied following exsanguination. Both tibiofibular bones are removed by dissection and all soft tissue is stripped. Bones from 5 - 6 rats for each group are stored in 70% ethanol for histological analysis, and bones from another 5 - 6 rats for each group are stored in a buffered Ringer's solution (+4°C, pH 7.4) for radiographs and biomechanical testing.

Histological Analysis: The methods for histologic analysis of fractured bone have been previously published by Mosekilde and Bak (The Effects of Growth Hormone on Fracture Healing in Rats: A Histological Descroption. Bone, 14:19-27, 1993). Briefly, the fracture side is sawed 8 mm to each side of the fracture line, embedded undecalcified in methymethacrylate, and frontal sections are cut on a Reichert-Jung Polycut microtome (8 µm thick). Masson-Trichrome stained mid-frontal sections (including both tibia and fibula) are used for visualization of the cellullar and tissue response to fracture healing with and without treatment. Sirius red stained sections are used to demonstrate the characterisitics of the callus structure and to differentiate between woven bone and lamellar bone at the fracture site. The following measurements are performed: (1) fracture gap - measured as the shortest distance between the cortical bone ends in the fracture, (2) callus length and callus diameter, (3) total bone volume area of callus, (4) bony tissue per tissue area inside the callus area, (5) fibrous tissue in the callus, and (6) cartilage area in the callus.

Biomechanical Analysis: The methods for biomechanical analysis have been previously published by Bak and Andreassen (The Effects of Aging on Fracture Healing in Rats. Calcif Tissue Int 45:292-297, 1989). Briefly, radiographs of all fractures are taken prior to the biomechanical test. The mechanical properties of the healing fractures are analyzed by a destructive three- or four-point bending procedure. Maximum load, stiffness, energy at maximum load, deflection at maximum load, and maximum stress are determined.

### Protocol for Effects On Bone Healing After Administration of Combinations of Prostaglandin Agonist and HMG-Co Reductase Inhibitors ― Dog Model

An ulnar segmental defect model is used to evaluate bone healing in 11 ± 1 kg beagle male dogs, 13 months old (as described by Cook et al. Use of an Osteoinductive Biomaterial (rhOP-1) in Healing large Segmental Bone Defects J. of Orthopaedic Trauma 12, 407-412, 1998.). Dogs are treated with antiparasitics one week before surgery.

Animals are also given 250 mg cefuroxime, by intravenous injection, one hour preoperatively and once more perioperatively if the operation lasts more than 2 hours.

Dogs are divided into the following groups of eight animals.

### Group A:

For local administration, carrier for the prostaglandin agonist and carrier for the HMG-CoA reductase inhibitor is injected in the defect area. The periosteum is then stitched together.

For systemic administration, carrier for the prostaglandin agonist and carrier for the HMG-CoA reductase inhibitor are given daily by oral gavage.

### Group B:

For local administration, carrier containing the prostaglandin agonist to be tested and the HMG-CoA reductase inhibitor to be tested is applied into the defect area and the periosteum is stitched together.

For systemic administration, carrier containing the prostaglandin agonist to be tested and carrier containing the HMG-CoA reductase inhibitor to be tested are given daily by oral gavage.

With the animal under general anesthesia, the foreleg is prepared and draped in sterile fashion. A lateral incision approximately 6 cm in length is made and the ulna is exposed extraperiostally. Periosteum is cut and moved to the proximal and distal parts of the incision. Then a 1.5 cm segmental defect is made in the midulna using a pendular saw. The radius and the remaining interosseal membrane are left intact. The defect site is irrigated with saline to remove bone debris. The site is then filled with the carrier as described above. The soft tissues are meticulously closed in layers to aid the carrier.

Following surgery animals will be allowed full weight-bearing activity, and water and food ad libitum. Radiographs of the forelimbs will be obtained immediately following surgery and every two weeks thereafter until the termination of the study. Radiographs will be graded on a 0 to 6 scale (Table 1).

**Table 1.**

| Radiographic Grading Scale | |
|---|---|
| Grade 0 | no change from immediate postoperative appearance |
| Grade 1 | trace of radiodense material in defects |
| Grade 2 | flocculent radiodensity with flecks of calcification and no defect bridging |
| Grade 3 | defect bridged at at least one point with material of nonuniform radiodensity |
| Grade 4 | defect bridged in medial and lateral sides with material of uniform radiodensity, cut ends of cortex remain visible |
| Grade 5 | same as Grade 3, at least one of four cortices obscured by new bone |
| Grade 6 | defect bridged by uniform new bone, cut ends of cortex not seen |

Along with radiographic evidence of healing histological analysis and biomechanical testing as described by Cook S. D. et al. Journal of Orthopedic Trauma, 12, 407-412, 1998 will be used to evaluate the efficacy of treatment.

Administration of the compound combinations of this invention can be via any method which delivers the compounds systemically and/or locally (e.g., at the site of the bone fracture, osteotomy, or orthopedic surgery. These methods include oral routes, parenteral, intraduodenal routes, etc. Generally, the compounds of this invention are administered orally, but parenteral administration (e.g., intravenous, intramuscular, subcutaneous or intramedullary or on sites of bone fracture) may be utilized, for example, where oral administration is inappropriate for the instant target or where the patient is unable to ingest the compound or compounds being administered.

The compound combinations may be used for the treatment and promotion of healing of bone fractures and osteotomies by the local application (e.g., to the sites of bone fractures of osteotomies) of the compounds or compositions thereof. The compound combinations of this invention may be applied to the sites of bone fractures or osteotomies, for example, either by injection of the compounds in a suitable solvent (e.g., an oily solvent such as arachis oil) to the cartilage growth plate or, in cases of open surgery, by local application thereto of such compounds in a suitable carrier such as bone-wax, demineralized bone powder, polymeric bone cements, bone sealants, etc. Alternatively, local application can be achieved by applying a solution or dispersion of the compounds in a suitable carrier onto the surface of the area being treated, or incorporating them into solid or semi-solid implants conventionally used in orthopedic surgery, such as Dacron®-mesh, gel-foam and kiel bone, or prostheses.

The compound combinations of this invention can be co-administered simultaneously or sequentially in any order, or as a single pharmaceutical composition.

The amount and timing of compounds to be administered will be dependent on the subject being treated, on the severity of the affliction, on the manner of administration and on the judgement of the prescribing physician. Thus, because of patient to patient variability, the dosages given below are a guideline and the physician may titrate doses of the compounds to achieve the treatment (e.g., bone mass augmentation) that the physician considers appropriate for the patient. In considering the degree of treatment desired, the physician must balance a variety of factors such as bone mass starting level, age of the patient, presence of preexisting disease, as well as presence of other diseases (e.g., cardiovascular diseases).

In general, an effective dosage for the prostaglandin agonists described above is in the range of 0.001 to 100 mg/kg/day, preferably 0.01 to 50 mg/kg/day. An effective dose of the statin compounds described above will be the range of about 0.1 mg/kg to about 1000 mg/kg/day, preferably about 1 mg/kg/day to about 200 mg/kg/day.

Prostaglandin agonists may be prepared by methods known in the art. For example, WO 98/27976, incorporated herein by reference, discloses the preparation of a number of prostaglandin agonists. Other prostglandin agonists may be prepared in accordance with the disclosures in U.S. Patent 3,932,389, U.S. Patent 3,982,016, U.S. Patent 4,000,309, U.S. Patent 4,018,892, U.S. Patent No. 4,097,601, U.S. Patent 4,132,847, U.S. Patent 4,219,483, U.S. Patent 4,621,100, U.S. Patent 5,216,183, U.S. Patent No. 5,703,108, U.S. Patent 6,124,314, WO 97/31640, WO 98/28264, WO 98/58911 and WO 99/19300, all of which are incorporated herein by reference. The prostaglandin agonists, 2-(3-{[2-(3,5-dichloro-phenoxy)-ethyl]-methanesulfonyl-amino}-propyl)-thiazole-4-carboxylic acid, 7-[(4-butyl-benzyl)-methanesulfonyl-amino]-heptanoic acid and 2-(3-{[3-(3-chloro-phenyl)-propyl]-methanesulfonyl-amino}-propyl)-thiazole-4-carboxylic acid may be prepared in accordance with the methods disclosed in WO 98/28264. The prostaglandin agonists, (3-(((2-(3,5-dichloro-phenoxy)-ethyl)-(pyridine-3-sulfonyl)-amino)-methyl)-phenoxy)-acetic acid; (3-(((4-dimethylaminobenzyl)-(pyridine-3-sulfonyl)-amino)-methyl)-phenoxy)-acetic acid; and (3-(((4-tert-butyl-benzyl)-(pyridine-3-sulfonyl)-amino)-methyl)-phenoxy)-acetic acid may be prepared in accordance with the methods disclosed in WO 99/19300.

HMG-CoA reductase inhibitors may be readily prepared by processes known in the chemical arts. Mevastatin, lovastatin, pravastatin, velostatin, simvastatin, fluvastatin, cerivastatin and mevastatin, dalvastatin and fluindostatin may be made in accordance with the process set forth in U.S. Patent No. 3,983,140, U.S. Patent No. 4,231,938, U.S. Patent No. 4,346,227, U.S. Patent No. 4,448,784, U.S. Patent No. 4,450,171, U.S. Patent No. 4,739,073, U.S. Patent No. 5,177,080, U.S. Patent No. 5,177,080, European Patent Application No. 738,510 A2 and European Patent Application No. 363,934 A1 respectively, which are all incorporated herein by reference.

Atorvastatin may readily be prepared as described in U.S. Patent No. 4,681,893, which is incorporated herein by reference. The hemicalcium salt of atorvastatin, which is currently sold as Lipitor®, may readily be prepared as described in U.S. Patent No. 5,273,995, which is incorporated herein by reference. Other pharmaceutically-acceptable cationic salts of atorvastatin may be readily prepared by reacting the free acid form of atorvastatin with an appropriate base, usually one equivalent, in a co-solvent.

The compound combinations of the present invention are generally administered in the form of a pharmaceutical composition comprising at least one of the compounds of this invention together with a pharmaceutically acceptable carrier, vehicle or diluent. Thus, the compounds of this invention can be administered individually or together, locally or systemically, in any conventional oral, parenteral, rectal or transdermal dosage form.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington: The Science and Practice of Pharmacy, Mack Publishing Company, Easter, Pa., 19th Edition 1995.

Pharmaceutical compositions according to the invention may contain 0.1%-95% of the compound combinations of this invention, preferably 1%-70%. In any event, the composition or formulation to be administered will contain a quantity of the compound combinations of this invention in an amount effective to provide the desired bone growth enhancement effect.

Since the present invention has an aspect that relates to the augmentation of bone growth by treatment with a combination of active ingredients which may be administered separately, the invention also relates to combining separate pharmaceutical compositions in kit form. The kit comprises a container for containing the separate compositions such as a divided bottle or a divided foil packet, however, the separate compositions may also be contained within a single, undivided container. Typically the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

An example of such a kit is a so-called blister pack. Blister packs are well known in the packaging industry and are being widely used for the packaging of pharmaceutical unit dosage forms (tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via said opening.

It may be desirable to provide a memory aid on the kit, e.g., in the form of numbers next to the tablets or capsules whereby the numbers correspond with the days of the regimen which the dosage form so specified should be ingested. Another example of such a memory aid is a calendar printed on the card as follows "First Week, Monday, Tuesday, ...etc., Second Week, Monday, Tuesday,...etc." Other variations of memory aids will be readily apparent. A "daily dose" can be a single tablet or capsule or several tablets or capsules to be taken on a given day. Also, a daily dose of the first compound can consist of one tablet or capsule while a daily dose of the second compound can consist of several tablets or capsules and vice versa. The memory aid should reflect this.

In another specific embodiment of the invention, a dispenser designed to dispense the daily doses one at a time in the order of their intended use is provided. Preferably, the dispenser is equipped with a memory-aid, so as to further facilitate compliance with the regimen. An example of such a memory-aid is a mechanical counter which indicates the number of daily doses that has been dispensed. Another example of such a memory-aid is a battery-powered microchip memory coupled with a liquid crystal readout, or audible reminder signal which, for example, reads out the date that the last daily dose has been taken and/or reminds one when the next dose is to be taken.

## Claims

1. A pharmaceutical composition comprising a therapeutically effective amount of a first compound, said first compound being a prostaglandin agonist, or a prodrug thereof or a pharmaceutically acceptable salt of said compound or said prodrug, and a therapeutically effective amount of a second compound, said second compound being a HMG-CoA reductase inhibitor, or a prodrug thereof or a pharmaceutically acceptable salt of said compound or said prodrug, together with a pharmaceutically acceptable vehicle, carrier or diluent.

2. A pharmaceutical composition of claim 1 wherein said first compound is selected from PGD₁, PGD₂, PGE₂, PGE₁, PGF₂ and PGF_{2α}.

3. A pharmaceutical composition of claim 1 wherein said first compound is selected from a selective EP₁, EP₂, EP₃ and EP₄ agonist.

4. A pharmaceutical composition of claim 1 wherein said first compound is selected from a selective EP₂ agonist, a selective EP₄ agonist and an EP₂/EP₄ agonist.

5. A pharmaceutical composition of claim 1 wherein said first compound is selected from:
2-(3-{[2-(3,5-dichloro-phenoxy)-ethyl]-methanesulfonyl-amino}-propyl)-thiazole-4-carboxylic acid;
2-(3-{[3-(3-chloro-phenyl)-propyl]-methanesulfonyl-amino}-propyl)-thiazole-4-carboxylic acid;
(3-(((4-tert-butyl-benzyl)-(pyridine-3-sulfonyl)-amino)-methyl)-phenoxy)-acetic acid;
(3-(((2-(3,5-dichloro-phenoxy)-ethyl)-(pyridine-3-sulfonyl)-amino)-methyl)-phenoxy)-acetic acid;
(3-(((4-dimethylamino-benzyl)-(pyridine-3-sulfonyl)-amino)-methyl)-phenoxy)-acetic acid; and
7-[(4-butyl-benzyl)-methanesulfonyl-amino]-heptanoic acid, prodrugs thereof and pharmaceutically acceptable salts of said compounds and said prodrugs.

6. A pharmaceutical composition of claim 1 wherein said second compound is selected from mevastatin, lovastatin, pravastatin, velostatin, simvastatin, fluvastatin, cerivastatin, dalvastatin, fluindostatin and atorvastatin, prodrugs thereof and pharmaceutically acceptable salts of said compounds or said prodrugs.

7. The pharmaceutical composition of claim 1 wherein said second compound is atorvastatin, prodrug thereofs and pharmaceutically acceptable salts of said compound and said prodrugs.

8. The pharmaceutical composition of claim 5 wherein said second compound is atorvastatin, prodrugs thereof and pharmaceutically acceptable salts of said compounds and said prodrugs.

9. A method of promoting bone growth comprising administering to a mammal:
a therapeutically effective amount of a first compound, said first compound being a prostaglandin agonist, prodrugs thereof and pharmaceutically acceptable salts of said prostaglanidin agonist and said prodrugs; and
a therapeutically effective amount of a second compound, said second compound being a HMG-CoA reductase inhibitor, prodrugs thereof and pharmaceutically acceptable salts of said inhibitor and said prodrugs.

10. The method of claim 9 wherein said first compound is selected from PGD₁, PGD₂, PGE₂, PGE₁, PGF₂, and PGF_{2α}.

11. The method of claim 10 wherein said first compound is selected from a selective EP₂ agonist, a selective EP₄ agonist and an EP₂/EP₄ agonist.

12. The method of claim 11 wherein said first compound is selected from:
2-(3-{[2-(3,5-dichloro-phenoxy)-ethyl]-methanesulfonyl-amino}-propyl)-thiazole-4-carboxylic acid;
2-(3-{[3-(3-chloro-phenyl)-propyl]-methanesulfonyl-amino}-propyl)-thiazole-4-carboxylic acid;
(3-(((4-tert-butyl-benzyl)-(pyridine-3-sulfonyl)-amino)-methyl)-phenoxy)-acetic acid;
(3-(((2-(3,5-dichloro-phenoxy)-ethyl)-(pyridine-3-sulfonyl)-amino)-methyl)-phenoxy)-acetic acid;
(3-(((4-dimethylamino-benzyl)-(pyridine-3-sulfonyl)-amino)-methyl)-phenoxy)-acetic acid; and
7-[(4-butyl-benzyl )-methanesulfonyl-amino]-heptanoic acid, prodrugs thereof and pharmaceutically acceptable salts of said compounds and said prodrugs.

13. The method of claim 9 wherein said second compound is atorvastatin, prodrugs thereof and pharmaceutically acceptable salts of said compound and said prodrugs.

14. The method of claim 12 wherein said second compound is atorvastatin calcium.

15. A kit comprising:
a. an amount of a first compound, said first compound being a prostaglandin agonist, prodrugs thereof and pharmaceutically acceptable salts of said compound and said prodrugs in a first dosage form;
b. an amount of a second compound, said second compound being a HMG-CoA reductase inhibitor, prodrugs thereof and pharmaceutically acceptable salts of said compound and said prodrugs in a second dosage form; and
c. a container.

16. A pharmaceutical composition comprising, separately or together, a prostaglandin agonist or a prodrug thereof or a pharmaceutically acceptable salt of said prostaglandin agonist or said prodrug and a HMG-CoA reductase inhibitor or a prodrug thereof or a pharmaceutically acceptable salt of said inhibitor or said prodrug for simultaneous, sequential or separate administration.

17. A pharmaceutical composition according to claim 16 comprising a mixture of a prostaglandin agonist or a prodrug thereof or a pharmaceutically acceptable salt of said prostaglandin agonist or said prodrug and a HMG-CoA reductase inhibitor or a prodrug thereof or a pharmaceutically acceptable salt of said inhibitor or said prodrug, together with a pharmaceutically acceptable vehicle, carrier or diluent.

18. The use of a prostaglandin agonist, prodrug thereof, pharmaceutically acceptable salt of said prostaglandin agonist or said prodrug and a HMG-CoA reductase inhibitor, prodrug thereof, pharmaceutically acceptable salt of said inhibitor or said prodrug in the manufacture of a medicament for the treatment of a mammal requiring promotion of bone growth.

19. A kit comprising a prostaglandin agonist or a prodrug thereof or a pharmaceutically acceptable salt of said prostaglandin agonist or said prodrug and a HMG-CoA reductase inhibitor or a prodrug thereof or a pharmaceutically acceptable salt of said inhibitor or said prodrug, in separate dosage forms, said dosage forms being suitable for simultaneous, sequential or separate administration.
